# DEMANDE DE BREVET EUROPEEN

(11) **EP 2 719 732 A1**
(43) Date de publication de la demande: **16.04.2014**
(21) Numéro de dépôt: 13004805.1
(22) Date de dépôt: 05.10.2013
(51) Int. Cl.: C09D 7/12, A61L 29/08, B05D 5/08, C08L 75/14

(54) **Produit de revêtement d'un corps relativement solide pour en favoriser le glissement et corps recouvert de ce produit**

(30) Priorité: 12.10.2012 FR 1202730
(71) Demandeur: Stsat AG, 6060 Sarnen (CH)
(72) Inventeur: Nouzies, Didier, 93460 Gournay-sur-Marne (FR)
(74) Mandataire: Flavenot, Bernard

(57) **Abrégé**

La présente invention concerne les produits de revêtement d'un corps relativement solide pour en favoriser le glissement en milieu liquide.

Le produit de revêtement selon l'invention est essentiellement caractérisé par le fait qu'il comporte un ensemble comprenant un liant sous la forme d'un prépolymère d'uréthane-acrylate, un polymère hydrophile constitué par au moins l'un des composés suivants : polyvinylpyrrolidone (PVP), polyéthylèneglycol (PEG), carboxyméthylcellulose (CMC), alginate, un composé pour améliorer l'osmolalité et un solvant, et des nanoparticules en état solide en dispersion dans cet ensemble.

La présente invention concerne aussi les corps solides pour application médicale recouverts avec le produit de revêtement défini ci-dessus.

## Description

La présente invention concerne les produits de revêtement d'un corps relativement solide pour en favoriser le glissement en milieu liquide, qui trouvent une application dans le domaine des instruments thérapeutiques ou de diagnostic réalisés en matière plastique, comme du PVC, du polyuréthane, ou à partir de métal ou de verre, quand ces instruments sont destinés à être introduits dans une cavité corporelle, comme par exemple des cathéters, des fils de guidage, des drains de plaies, des fibres optiques, etc.

La présente invention concerne aussi les instruments qui sont recouverts de ces produits.

En ce qui concerne les cathéters, la présence du revêtement diminuant le frottement rend la surface du cathéter beaucoup plus glissante lorsqu'il est immergé dans une solution aqueuse éventuellement saline avant son insertion dans une cavité corporelle, ou lorsqu'il est mis en contact avec un liquide corporel aqueux au niveau de l'insertion dans la cavité corporelle comme un vaisseau sanguin ou analogue.

Ainsi, l'inconfort ressenti par le patient lorsque le cathéter est inséré dans la cavité corporelle et en est retiré est considérablement réduit, et en outre le risque d'endommagement des tissus sensibles à l'occasion de l'utilisation du cathéter est considérablement réduit. Le cathéter profite en outre de bien meilleures performances de navigation grâce à l'amélioration de la glisse apportée par le revêtement.

Il est connu, notamment par le EP-A-0591091, de tels produits qui présentent l'effet lubrifiant souhaité grâce à une libération prolongée de polymères hydrophiles.

Lorsqu'il s'agit d'un instrument à insérer dans le corps humain, et que l'on doit pouvoir enlever après une longue période d'insertion, il est d'une importance primordiale qu'une quantité suffisante de polymère hydrophile soit libérée au cas où l'on souhaite un effet lubrifiant à la fois pendant l'insertion de l'instrument dans la cavité et pendant son enlèvement ultérieur hors de celle-ci.

On peut aussi citer, comme art antérieur à l'invention, les brevets ainsi que divers articles scientifiques qui sont référencés dans le EP-A-0591091 mentionné ci-dessus. Il peut aussi être cité les documents suivants : le WO93/17077, le US 3293203 et le US 2009/032499.

Or, la production de revêtements hydrophiles pose le problème qui réside dans le fait que ces revêtements doivent contenir une quantité considérable de polymère hydrophile afin de posséder un caractère hydrophile suffisant, et dans le fait que les polymères hydrophiles sont difficiles à maintenir sur la surface d'articles solides, notamment lorsque ces articles sont réalisés en des matériaux hydrophobes, tels que le PVC ou le polyuréthane.

De plus, il est habituellement nécessaire d'utiliser un liant sous la forme d'un matériau hydrophobe, mais ce liant diminue encore le caractère hydrophile de la surface ainsi que sa capacité à devenir glissante et lubrifiante en situation humide.

Malgré les progrès qui ont été fait ces dernières années, il existe toujours des problèmes au moins dans certaines circonstances particulières.

Aussi, la présente invention a-t-elle pour but de réaliser un produit de revêtement d'un corps relativement solide pour en favoriser le glissement en milieu liquide et pour obtenir un glissement amélioré par rapport à celui permis avec les produits de l'art antérieur qui ont le même but.

Plus précisément, la présente invention a pour objet un produit de revêtement d'un corps relativement solide pour en favoriser le glissement en milieu liquide, comme défini dans les revendications annexées.

La présente invention aussi pour objet un corps solide pour application médicale recouvert avec un produit de revêtement tel que défini ci-dessus, notamment de façon préférentielle, un corps tel qu'un cathéter.

D'autres caractéristiques et avantages de la présente invention apparaîtront au cours de la description suivante.

La présente invention a pour but de réaliser un perfectionnement aux produits de revêtement d'un corps relativement solide pour en favoriser le glissement en milieu liquide.

Le produit de revêtement selon l'invention comporte un ensemble comprenant au moins un liant sous la forme d'un prépolymère d'uréthane-acrylate, un polymère hydrophile constitué par au moins l'un des composés suivants : polyvinylpyrrolidone (PVP), polyéthylèneglycol (PEG), carboxyméthyl-cellulose (CMC), alginate, un composé pour améliorer l'osmolalité et un solvant.

Selon une caractéristique de l'invention, le produit de revêtement comporte en outre des nanoparticules en état solide en dispersion dans l'ensemble défini ci-dessus.

Ces nanoparticules permettent d'améliorer le glissement du corps recouvert par le produit de revêtement selon l'invention, par le fait qu'elles ont une fonction qui pourrait être qualifiée de rotationnelle sur les parois sur lesquelles le corps revêtu de ce produit doit se translater, par exemple les parois d'un vaisseau sanguin parcouru par un cathéter ou analogue.

De façon préférentielle, ces nanoparticules sont en des matériaux biocompatibles, comme du poly-éther-éther-cétone (PEEK).

Cependant, de très bons résultats peuvent être obtenus, au moins pour certaines applications médicales, quand elles sont, par exemple, en polytetrafluoroéthylène.

De façon avantageuse, ces nanoparticules ont des dimensions au plus égales à 100⁻⁹ m.

De façon préférentielle, elles sont en dispersion dans l'ensemble défini ci-dessus, à un taux en poids au plus égal à 10 %, optionnellement au moins égal à 3%.

De façon préférée, du fait de sa simplicité et de son coût de revient, le solvant est de l'eau en quantité d'au moins 20% en poids dans l'ensemble.

De façon encore plus préférentielle, les nanoparticules en état solide sont sensiblement sphériques ou sensiblement cylindriques de révolution pour faciliter leur fonction rotationnelle.

La présente invention a aussi pour objet un corps solide pour application médicale recouvert avec un produit de revêtement tel que défini ci-dessus.

Ce corps est par exemple un cathéter, un fil de guidage, une fibre optique ou analogue, un drain de plaie.

## Revendications

1. Produit de revêtement d'un corps relativement solide pour en favoriser le glissement en milieu liquide, ledit produit de revêtement comportant un ensemble comprenant un liant sous la forme d'un prépolymère d'uréthane-acrylate, un polymère hydrophile constitué par au moins l'un des composés suivants : polyvinylpyrrolidone (PVP), polyéthylèneglycol (PEG), carboxyméthylcellulose (CMC), alginate, un composé pour améliorer l'osmolalité et un solvant, **caractérisé par le fait qu'**il comporte en outre des nanoparticules en état solide en dispersion dans ledit ensemble.

2. Produit de revêtement selon la revendication 1, **caractérisé par le fait que** lesdites nanoparticules ont des dimensions au plus égales à 100⁻⁹m.

3. Produit de revêtement selon la revendication 2, **caractérisé par le fait que** lesdites nanoparticules sont en dispersion dans ledit ensemble à un taux en poids au plus égal à 10%.

4. Produit de revêtement selon la revendication 3, **caractérisé par le fait que** lesdites nanoparticules sont en dispersion dans ledit ensemble à un taux en poids au moins égal à 3%.

5. Produit de revêtement selon l'une des revendications précédentes, **caractérisé par le fait que** ledit solvant est de l'eau en quantité d'au moins 20% en poids dans ledit ensemble.

6. Produit de revêtement selon l'une des revendications précédentes, **caractérisé par le fait que** lesdites nanoparticules en état solide sont sphériques.

7. Produit de revêtement selon l'une des revendications 1 à 6, **caractérisé par le fait que** lesdites nanoparticules sont du polytetrafluoroéthylène.

8. Corps solide pour application médicale recouvert avec un produit de revêtement selon l'une des revendications 1 à 7.

9. Corps solide selon la revendication 8, **caractérisé par le fait qu'**il est constitué de l'un des corps suivants : un cathéter, un fil de guidage, une fibre optique, un drain de plaie.
